(19) Europäisches Patentamt
European Patent Office
Office européen des brevets

(11) Veröffentlichungsnummer: **0 518 457 A1**

(12) # EUROPÄISCHE PATENTANMELDUNG

(21) Anmeldenummer: **92250152.3**

(22) Anmeldetag: **15.06.92**

(51) Int. Cl.5: **C07D 403/06**, C07D 233/60, C07D 249/18, C07D 235/06, A61K 31/395, A61K 31/41, C07D 249/04, C07D 249/08

(30) Priorität: **14.06.91 DE 4120107**

(43) Veröffentlichungstag der Anmeldung:
**16.12.92 Patentblatt 92/51**

(84) Benannte Vertragsstaaten:
**AT BE CH DE DK ES FR GB GR IT LI LU NL PT SE**

(71) Anmelder: **SCHERING AKTIENGESELLSCHAFT**
**Müllerstrasse 170/178 Postfach 65 03 11**
**W-1000 Berlin 65(DE)**

(72) Erfinder: **Strehlke, Peter, Dr.**
**Droysenstrasse 10a**
**W-1000 Berlin 12(DE)**
Erfinder: **Bohlmann, Rolf, Dr.**
**Kühler Weg 6a**
**W-1000 Berlin 19(DE)**
Erfinder: **Muhn, Peter, Dr.**
**Am Waldidyll 23**
**W-1000 Berlin 28(DE)**
Erfinder: **Nishino, Yukishige, Dr.**
**Landzendorfer Weg 14**
**W-1000 Berlin 22(DE)**
Erfinder: **Schneider, Martin, Dr.**
**Schluchseestrasse 6a**
**W-1000 Berlin 28(DE)**

(54) **Bicyclisch substituierte Vinylimidazole, -triazole und -tetrazole.**

(57) Die Erfindung betrifft bicyclisch substituierte Vinylimidazole, -triazole und -tetrazole der allgemeinen Formel I

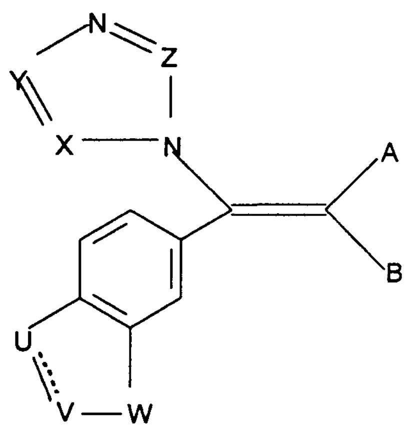

(I),

mit verbesserter aromatasehemmender Wirkung.

EP 0 518 457 A1

Die vorliegende Erfindung betrifft bicyclisch substituierte Vinylimidazole, -triazole und -tetrazole der allgemeinen Formel I

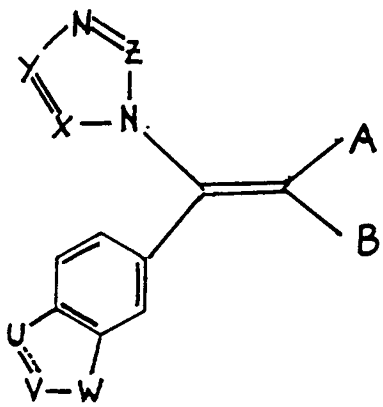

(I),

worin

X, Y und Z gemeinsam mit den beiden Stickstoffatomen einen Imidazol-, Triazol- oder Tetrazolrest bilden, A und B gleich oder verschieden sind und je ein Wasserstoffatom, eine gerad- oder verzweigtkettige $C_1$-$C_{12}$ - Alkylgruppe oder eine gegebenenfalls substituierte Arylgruppe oder entweder

A oder B ein Wasserstoffatom und der jeweils andere Substituent B oder A eine gegenenfalls veresterte Caboxylgruppe, eine gege-benenfalls substituierte Carbonsäureamidgruppe, eine Alkyl-oder Arylketongruppe oder eine Nitrilgruppe oder A und B gemeinsam einen Cycloalkyliden oder Polycycloalkylidenrest,die jeweils ein- oder mehrfach durch $C_1$ - $C_8$ - Alkylgruppen oder Halogenatome substituiert sein können, und U entweder den Rest >CH-OH oder die Gruppe >C=D, wobei D für ein Sauerstoffatom oder für einen Rest =N-$OR^1$ sowie -V-W- für einen Al-kylenrest -$(CH_2)_n$- mit n = 2, 3 oder 4 stehen, oder

U die doppelt an V gebundene Gruppe ⋗C-$R^2$, wobei V den Rest ⋗C-$R^3$ und W den Rest >N-$R^4$ darstellen, oder U ein doppelt an V gebundenes Stickstoffatom, wobei V dann ebenfalls ein Stickstoffatom oder die Gruppierung ⋗C-$R^5$ und W die Gruppe >N-$R^6$ darstellen mit $R^1$, $R^2$, $R^3$, $R^4$, $R^5$ und $R^6$ jeweils in der Bedeutung eines Wasserstoffatoms oder einer gerad- oder verzweigtkettigen $C_1$-$C_{12}$ - Alkylgruppe, bedeuten.

Durch diese für U, V und W angegebenen Definitionen ergeben sich damit für den bicyclischen Substituenten an der vinylischen Doppelbindung folgende Ringsysteme:

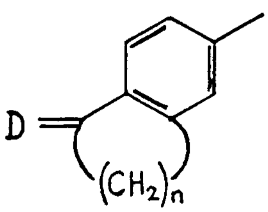

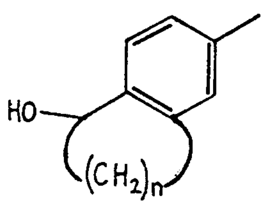

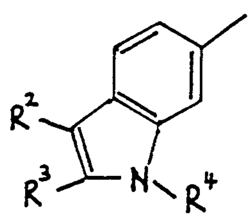

(Indol)

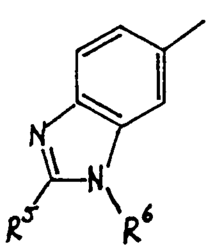

(Benzimidazol)

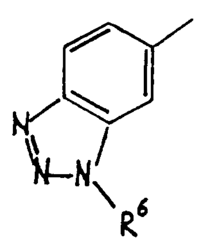

(Benzotriazol)

Wenn A oder B einen Arylrest darstellen, so soll dies vorzugsweise ein Phenyl-, Naphthyl- oder Heteroarylrest wie z. B. ein Thiophen-, Furan-, Pyridin-, Thiazol-, Oxazol- oder Diazinring sein, der gegebenenfalls durch ein oder mehrere Niedrigalkylreste (1 bis 4 C-Atome) oder Halogenatome, vorzugsweise Fluor, Chlor oder Brom substituiert sein kann.

Steht A oder B für eine veresterte Carboxylgruppe, so ist diese in erster Linie mit einem gerad- oder verzweigtkettigen oder cyclischen O-Alkylrest mit bis zu 10 Kohlenstoffatomen, mit einem O-Arylrest, wobei Aryl ein gegebenenfalls durch ein oder mehrere Niedrikalkylgruppen (1-4 Kohlenstoffatome) oder Halogenatome (F, C1, Br, I) substituierter Phenyl- oder Naphthylrest ist oder einem O-Aralkylrest, wobei in diesem das Aryl- und Alkylfragment die vorstehend angegebene Bedeutung haben, verestert.

Insbesondere bevorzugt sind hierbei der Methoxy-, Ethoxy-, Propoxy-, Isopropoxy, Isobutoxy-, tert.-Butoxy-, Cyclohexyloxy-, Cyclopentyloxy-, Phenoxy- oder 2,6-Dichlorphenoxyrest.

Handelt es sich bei A oder B um eine substituierte Carbonsäureamidgruppe ist diese vor allem mit einem oder zwei , im letzten Fall gleichen oder verschiedenen Resten substituiert. Diese Reste können gerad- oder verzweigtkettige Alkylreste mit 1 bis 10 Kohlenstoffatomen, oder gegebenenfalls durch Alkylgruppen oder Halogenatome substituierte Arylreste mit 6 bis 10 Kohlenstoffatomen sein. Ferner kann das amidische Stickstoffatom auch Teil eines 5- bis 8-gliedrigen Ringes sein, der auch >N-$R^7$ mit $R^7$ in der Bedeutung eines Wasserstoffatoms oder einer gerad- oder verzweigtkettigen Alkylgruppe mit 1 bis 6 Kohlenstoffatomen, O oder S als Ringglied enthalten kann.

Ganz besonders hervorzuheben ist die Substitution der Carbonsäureamidgruppe mit einem Methyl-, Ethyl-, Propyl-, tert.-Butyl-, Phenyl-, Benzylrest, zwei Methyl-, Ethyl-, Propylresten, einem Phenyl- und einem Methyl-, einem Phenyl- und einem Ethyl- sowie einem Benzyl- und einem Methylrest oder ein gemeinsam mit dem amidischen Stickstoffatom gebildeter Pyrrolidin-, Piperidin-, Piperazin-, N-Methylpiperazin-, Morpholin- oder Thiomorpholinring.

Als Alkylketongruppe A oder B ist der Rest -CO-$R^8$, wobei $R^8$ einen gerad- oder verzweigtkettigen Alkylrest mit 1 bis 10 oder einen Cycloalkylrest mit 3 bis 12 Kohlenstoffatomen bedeutet und als Arylketongruppe der Rest -CO-$R^9$, wobei $R^9$ einen gegebenenfalls durch Alkyl-, Halogen-, Hydroxy- oder Alkoxyreste substituierten Phenyl-, Naphtyl- oder Heteroarylrest wie z. B. einen Thiophen-, Furan-, Pyridin-, Thiazol-, Oxazol- oder Diazinring bedeutet, bevorzugt.

Insbesondere ist $R^8$ ein Methyl-, Ethyl-, Propyl-, Isopropyl-, Butyl-, Isobutyl-, Pentyl-, Isopentyl-, Neopentyl-, Cyclopentyl- oder Cyclohexylrest und $R^9$ ein Phenyl-, Hydroxyphenyl-, Methoxyphenyl- oder Chlorophenylrest.

Die Verbindungen der allgemeinen Formel I sind Inhibitoren der Estrogenbiosynthese (Aromatasehemmer). Sie sind damit zur Behandlung von Krankheiten geeignet, die durch Estrogene bedingt oder von Estrogenen abhängig sind. So sind sie geeignet zur Behandlung von estrogeninduzierten oder -stimmulierten Tumoren, wie zum Beispiel Mammakarzinom oder Prostatahyperplasie (The Lancet, **1984**, 1237-1239, J. Med. Chem. 33, **1990**, 2933).

Die erfindungsgemäßen Verbindungen sind auch wertvoll zur Beeinflussung der Fertilität. So kann eine männliche Infertilität, die aus erhöhten Estrogenspiegeln resultiert, mit den neuen Wirkstoffen behoben werden. Ferner können die Verbindungen bei Frauen im fortpflanzungsfähigen Alter als Antifertilitätsmittel verwendet werden, um Ovulationen durch Estrogenentzug zu hemmen.

Wahrscheinlich sind Aromatasehemmer auch zur Behandlung des drohenden Herzinfarktes geeignet, da erhöhte Estrogenspiegel beim Mann einem Herzinfarkt vorangehen können (US-Patent 4,289,762).

Bekannte, eine aromatasehemmende Wirkung aufweisende Substanzen sind neben Steroiden auch nichtsteroidale Substanzen, beispielsweise die in den europäischen Patentanmeldungen EP-A 0165777 bis 0165784 beschriebenen diversen Stickstoff-heterocyclen, die in J. Med. Chem. 1986, 29, Seiten 1362-1369 beschriebenen substituierten Glutarsäureimide, die in der europäischen Patentanmeldung EP 0165904 beschriebenen substituierten Imidazobenzole, die in der europäischen Patentanmeldung EP-A 0236940 beschriebenen heterocyclisch substituierten Toluolnitrile sowie die aus dem US-Patent US-A-4,728,465 hervorgehenden, einen gegebenenfalls substituierten Phenylring tragenden Imidazo- und 5,6,7,8-Tetrahydroimidazo[1,5a]pyridine, aus denen insbesondere das 5-(p-Cyanophenyl)-5,6,7,8-tetrahydroimidazo-[1,5 a]pyridin, Hydrochlorid als stark wirksamer Aromatasehemmer herausragt (Cancer Res. 48, S. 834-838, **1988**).

Die Verbindungen der vorliegenden Anmeldungen zeichnen sich gegenüber den bisher bekannten Verbindungen dadurch aus, daß sie das Enzymsystem der Aromatase stärker und zugleich selektiver hemmen. Die selektive Wirkung zeigt sich daran, daß andere Enzymsysteme in geringerem Umfang beeinträchtigt werden.

Die zu verabreichende Menge der Verbindungen schwankt innerhalb eines weiten Bereichs und kann jede wirksame Menge abdecken. In Abhängigkeit des zu behandelnden Zustands und der Art der

Verabreichung kann die Menge der verabreichten Verbindungen 0.0001-10 mg/kg Körpergewicht, vorzugsweise 0.001-1 mg/kg Körpergewicht, je Tag betragen.

Zur oralen Verabreichung kommen Kapseln, Pillen, Tabletten, Dragees usw. in Frage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten. Die einzelnen Dosierungseinheiten für die orale Applikation können beispielsweise 0,05-50 mg des Wirkstoffes (Aromatasehemmers) enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiergemisches verwendet. Als Beispiele für verwendete Öle sind zu nennen: olivenöl, Erdnußöl, Baumwollöl, Sojabohnenöl, Rizinusöl und Sesamöl.

Die Verbindungen lassen sich in Form einer Depotinjektion oder eines Implantatpräparates anwenden, die so formuliert sein können, daß eine verzögerte Wirkstoff-Freigabe ermöglicht wird.

Implantate können als inerte Materialien zum Beispiel biologisch abbaubare Polymere enthalten oder synthetische Silicone, wie zum Beispiel Siliconkautschuk. Die Wirkstoffe können außerdem zur perkutanen Applikation, zum Beispiel in Pflaster, eingearbeitet werden.

Die Erfindung betrifft somit auch pharmazeutische Präparate und die Verwendung der Verbindungen zur Herstellung dieser Präparate zur Behandlung von estrogen-bedingten Krankheiten.

Die Tumor-Hemmwirkung von Imidazolderivaten beruht auf einer Inhibierung P-450 abhängiger Enzymsysteme (vgl. z.B. J. P. Van Wanne und P. A. J. Janssen, J. Med. Chem. 32, 1988, 2231). Auch die Wirkung antifungaler Therapeutika aus der Reihe der Imidazol- und Triazolderivate beruht auf einer Blockade P-450-abhängiger biochemischer Reaktionen (loc. cit.). Ferner ist aus der Patentliteratur bekannt, daß Azol-Derivate sowohl antifungale als auch tumorhemmende Wirkung zugleich haben (vgl. hierzu EPA 0165777, Eli Lilly). Die erfindungsgemäßen Verbindungen sollten daher auch eine antifungale Wirkung gegen human-, tier- und pflanzenpathogene Keime aufweisen.

Die vorliegende Erfindung betrifft außerdem ein Verfahren zur Herstellung der Verbindungen der allgemeinen Formel I. Dabei wird entweder

a) eine Verbindung der allgemeinen Formel II

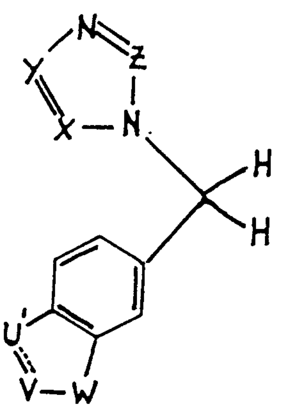

(II),

worin

V, W, X, Y und Z die in Formel I angegebene Bedeutung haben, U' den Rest >CH-OH oder >C=N-OR[1], wobei R[1] die in Formel I angegebene Bedeutung hat und die Hydroxygruppe in U' gegebenenfalls geschützt ist, bedeutet und die H-Atome der die beiden Ringsysteme überbrückenden Methylengruppe acide sind mit einem Keton der allgemeinen Formel III

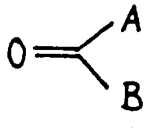

(III),

worin A und B die in Formel I angegebene Bedeutung haben, in Gegenwart einer Base zu einer Verbindung der allgemeinen Formel IV

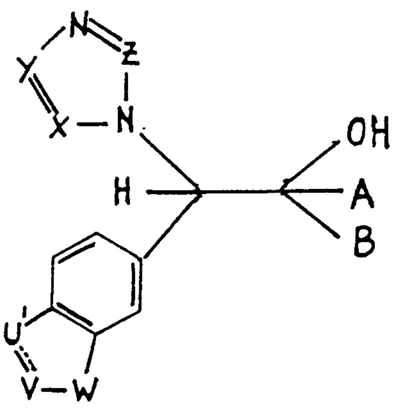

(IV),

umgesetzt und diese mit einem wasserabspaltenden Mittel, und nach gegebenenfalls erforderlicher Abspaltung der Schutzgruppe in U' in eine Verbindung der allgemeinen Formel I umgewandelt oder

b) eine Verbindung der allgemeinen Formel V

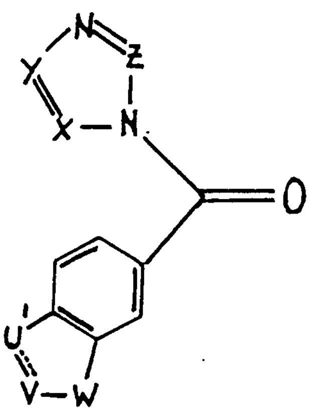

(V),

worin

V, W, X, Y und Z die in Formel I angegebene Bedeutung haben und U' für den Rest >CH-OH oder >C=N-OR[1], wobei $R^1$ die in Formel I angegebene Bedeutung hat mit einem Wittig-Reagens der allgemeinen Formel VI

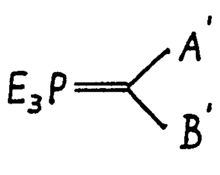

(VI),

worin A' oder B' ein Wasserstoffatom und der jeweils andere Substituent B' oder A' eine gegebenenfalls veresterte Carboxylgruppe, eine gegebenenfalls substituierte Carbonsäureamidgruppe, eine Alkyl- oder Arylketongruppe oder eine Nitrilgruppe sowie E einen gegebenenfalls mit einem oder mehreren gerad- oder verzweigtkettigen $C_1$-$C_6$-Alkylresten oder Halogenatomen substituierten Phenylrest oder einen gerad- oder verzweigt-kettigen $C_{1-6}$-Alkylrest bedeuten, in einem inerten Lösungsmittel zwischen Raumtemperatur und Siedetemperatur des verwendeten Lösungsmittels umgesetzt oder

c) eine Verbindung der allgemeinen Formel VII

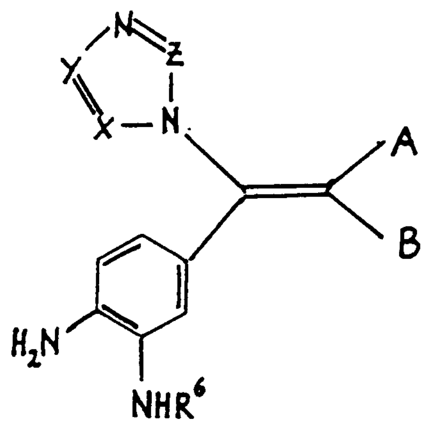

(VII),

worin A, B, X, Y, Z und $R^6$ die in Formel I angegebene Bedeutung haben, in an sich bekannter Weise mit einer Säure $R^5$-COOH oder einem Aldehyd $R^5$-CHO, worin $R^5$ die in Formel I angegebene Bedeutung hat, im Falle des Aldehyds unter milde oxidierenden Bedingungen, oder mit salpetriger Säure oder deren Estern umgesetzt.

Bei der erfindungsgemäßen Verfahrensvariante a) ist zu beachten, daß die entsprechenden Ausgangsverbindungen der allgemeinen Formel II in U', V und W keine aciden Wasserstoffatome aufweisen dürfen. Die Umsetzung mit einem Keton der allgemeinen Formel III erfolgt nach Deprotonierung der entsprechenden Verbindung der allgemeinen Formel II mit einem oder zwei Equivalenten einer starken Base wie z. B. Lithium-diisopropylamid, primären, sekundären oder tertiärem Butyllithium. Zwei Equivalente einer starken Base sind dann erforderlich, wenn Z für eine Methingruppe >CH steht, da dann zuerst deren Proton und erst anschließend ein Proton der die beiden Ringsysteme überbrückenden Methylengruppe abgespalten wird.

Die erhaltene Verbindung der Formel IV wird, gegebenenfalls nach Überführung der Hydroxygruppe in eine bessere Abgangsgruppe Ab, entweder durch Erhitzen oder durch Zugabe einer Base, gegebenenfalls unter zusätzlichem Erhitzen durch Abspaltung von HOH oder, falls die Hydroxygruppe in eine Abgangsgruppe Ab überführt wurde, durch Abspaltung von HAb in eine Verbindung der allgemeinen Formel I umgewandelt.

Vorzugsweise wird die Hydroxygruppe vor der Einführung der Doppelbindung in eine Chlorfunktion durch Umsetzung der Hydroxyverbindung beispielsweise mit Thionychlorid überführt. Die anschließende Abspaltung von Chlorwasserstoff kann dann einfach durch weiteres Erhitzen im Chlorierungsmittel bewerkstelligt werden.

Im allgemeinen ist es jedoch zweckmäßig, die Einführung der Doppelbindung durch Abspaltung von HOH oder HAb unter Einwirkung einer Base vorzunehmen. Als Base kommen neben anderen beispielsweise mäßig konzentrierte wäßrige Natronlauge oder Triethylamin infrage.

Die erfindungsgemäße Verfahrensvariante b) führt durch Reaktion eines Acylazols der allgemeinen Formel V mit einem Phosphoran der Formel VII zu den Verbindungen der allgemeinen Formel Ib (Wittig-Reaktion). Als inerte Lösungsmittel dienen beispielsweise Benzol, Toluol, Xylol, Chlorbenzol, Tetrahydrofuran, Dioxan, Acetonitril, Dimethylformamid oder Dimethylsulfoxid. Die Reaktionstemperatur wird vorzugsweise über 60 ° C gewählt.

Daß Azylazole mit einem Phosphoran im Sinne einer WittigReaktion reagieren, wird erstmals in der deutschen Patentanmeldung P 40 39 559.6 beschrieben. Da Acylazole gute Acylierungsmittel sind (Comprehensive Heterocyclic Chemistry, Eds. A. R. Katritzky, K. T. Potts, Pergamon Press **1984**, Band 4A, Seite 451 ff.), wäre auch bei der erfindungsgemäßen Reaktion b) eher eine Acylierung des Wittig-Reagenzes (Phosphoran) der allgemeinen Formel VI am Methylenkohlenstoffatom zu erwarten gewesen.

Die als Ausgangsprodukt benötigten Acylazole der allgemeinen Formel V werden in an sich bekannter Weise aus den entsprechenden Carbonsäuren der Formel

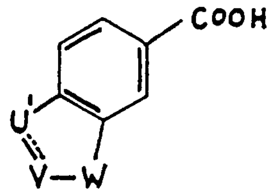

oder Derivaten dieser Carbonsäuren, vorzugsweise den Säurechloriden, durch Umsetzung mit geeigneten Azolderivaten wie Carbonyl- oder Sulfinyldiazol, Trialkylsilylazol oder aber dem Azol selbst oder einem Metallsalz des Azols hergestellt. Die genannten Umsetzungen werden vorzugsweise in inerten Lösemitteln wie Diethylether, Tetrahydrofuran, Dichlormethan oder Tolnol bei Temperaturen zwischen 0° und 50°C, in der Regel bei Raumtemperatur vorgenommen. Als Azol-Metallsalze kommen vorzugsweise Alkali- und Erdalkali- oder Silbersalze infrage.

Zur Synthese der Verbindungen der allgemeinen Formel VII als Ausgangsprodukte für die erfindungsgemäße Verfahrensvariante c) geht man aus von Vorstufen der allgemeinen
Formeln VIII oder IX

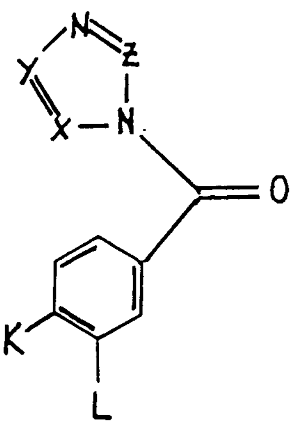

(VIII),

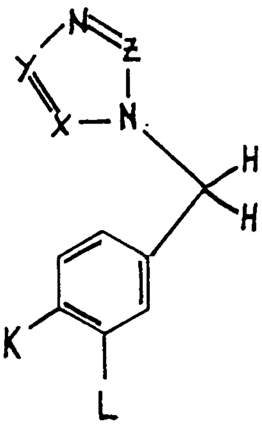

(IX),

worin K und L Gruppen darstellen, die sich einfach in Aminogruppen bzw. im Fall von L auch in eine Alkylaminogruppe umwandeln lassen. In erster Linie ist hier an Nitrogruppen zu denken, die sich nach Standardverfahren reduktiv in Aminogruppen überführen lassen. Ist K eine Nitrogruppe und L ein Fluoratom, so kann durch nucleophile Substitution das Fluoratom gegen eine Alkylaminogruppe ausgetauscht werden und man erhält nach Reduktion für K eine Amino-, für E aber eine Alkylaminogruppe - $NHR^6$.

Die Verbindungen der allgemeinen Formel VIII selbst, sind nach der bei der Beschreibung der Verfahrensvariante b) angegebenen Methode herstellbar aus den entsprechenden Carbonsäuren oder ihren Derivaten. Sie lassen sich analog Variante b) in die Verbindungen der allgemeinen Formel VII überführen. Verbindungen der allgemeinen Formel IX erhält man durch N-Alkylierung eines Azols mit dem entsprechenden Benzylhalogenid nach gängigen Verfahren. Die Umsetzung analog Variante a) führt dann zu den Verbindungen der allgemeinen Formel VII.

Die nachfolgenden Beispiele dienen der näheren Erläuterung der Erfindung.

## Beispiel 1

***3-(1-Imidazolyl)-3-(1-methyl-6-benzotriazolyl)-acrylsäure-tert.butylester***

Benzotriazol-5-carbonsäure-methylester wird mit Kaliumcarbonat/Methyljodid in Tetrahydrofuran methyliert. Das Produkt wird chromatographisch getrennt in:
1-Methyl-benzotriazol-5-carbonsäure-methylester und
1-Methyl-benzotriazol-6-carbonsäure-methylester,
Fp. 154-156°C.

1,8 g des 1-Methyl-benzotriazol-6-carbonsäure-methylesters werden mit 100 ml Kaliumhydroxid in Methanol (10 % G/V) 3 Stunden am Rückfluß gekocht. Nach Abziehen des Lösemittels wird in wenig Wasser gelöst und durch Zugabe von 6 M Salzsäure die Säure ausgefällt.

1,6 g der Säure werden mit 20 ml Thionylchlorid eine Stunde am Rückfluß gekocht. Nach vollständigem Eindampfen hinterbleibt das Säurechlorid als hellbraune Kristallmasse. Dieses wird in 60 ml Tetrahydrofuran gelöst und mit 1,4 g N-Trimethylsilylimidazol 0,5 Stunden gerührt. Nach Eindampfen des Lösemittels im Vakuum wird der Rückstand in 120 ml Tetrahydrofuran gelöst und mit 3,38 g tert. Butoxycarbonyltriphenylphosphoran 16 Stunden am Rückfluß gekocht. Man gibt 240 ml 1 M Salzsäure hinzu, extrahiert mit Ether, alkalisiert die Wasserphase mit Kaliumcarbonat und extrahiert erneut mit Essigester. Nach Trocknen der Essigesterphase ($Na_2SO_4$) und Eindampfen wird aus Ether kristallisiert. Man erhält 630 mg eines Z-/E-Gemisches der Titelverbindung. Durch Umkristallisieren aus Cyclohexan/Ethanol erhält man 320 mg des E-Isomers, Fp. 184-185° C.

## Beispiel 2

***6-[Cyclohexyliden-(1-imidazolyl)-methyl]-1-methyl-benzotriazol***

20 g 1-Methyl-benzotriazol-6-carbonsäuremethylester (siehe Beispiel 1) werden in 1 l Tetrahydrofuran gelöst und mit 4 g Lithiumaluminiumhydrid bei Raumtemperatur portionsweise versetzt. Nach 2,5 Stunden werden bei 0°C 200 ml Essigester zugetropft, der Niederschlag abgesaugt, das Filtrat getrocknet und eingedampft. Man erhält 18.2 g (1-Methyl-benzotriazol-6-yl)-methanol. 4,75 g davon werden in 125 ml Tetrachlorkohlenstoff und 44 ml Acetonitril gelöst und bei 0°C mit 12,6 g Triphenylphosphin versetzt. Nach 2,5 Stunden bei 0°C wird mit Essigester verdünnt, mit Wasser und Kochsalzlösung gewaschen, getrocknet ($Na_2SO_4$) und eingedampft. Der Rückstand wird an Kieselgel chromatographiert. Mit Hexan/Essigester (1:1) eluiert man 1,6 g 6-Chlormethyl-1-methyl-benzotriazol.

Dieses wird in 20 ml Dimethylformamid gelöst und nach Zugabe von 1,35 g Imidazol mit 576 mg Natriumhydrid (60%ig in Mineralöl) portionsweise versetzt. Nach einer Stunde bei Raumtemperatur wird mit Essigester verdünnt, mit Kochsalzlösung gewaschen, getrocknet, eingedampft und der Rückstand aus Essigester kristallisiert. Man erhält so 6-(1-Imidazolylmethyl)-1-methyl-benzotriazol, Fp.157°C.

1,9 g davon werden in 86 ml trockenem Tetrahydrofuran gelöst und bei -70° C mit 12,2 ml einer 1,4 M Lösung von tert. Butyllithium in Pentan versetzt. Nach 0,5 Stunden Rühren bei von -70°C auf -50°C ansteigender Temperatur werden bei -50°C 0,92 ml Cyclohexanon zugetropft. Nach einer Stunde bei -50°C wird festes Ammoniumchlorid und danach Wasser zugegeben und mit Essigester extrahiert. Die Essigesterphase wird mit Wasser gewaschen, getrocknet ($Na_2SO_4$) und eingedampft. Der Rückstand wird in 25 ml Methylenchlorid gelöst und bei 0°C mit 1,12 ml Thionylchlorid langsam versetzt. Nach einer Stunde Rühren bei Raumtemperatur wird mit Essigester verdünnt, dann bei 0°C mit 2M Natronlauge alkalisiert, eine Stunde nachgerührt und auf Wasser gegeben.

Nach Extraktion mit Essigester, Trocknen ($Na_2SO_4$) und Eindampfen erhält man 2,1 g eines Rohproduktes, das an 200 g Kieselgel chromatographiert wird. Es wird mit Methylenchlorid unter Zusatz von bis zu 15 % (V/V) steigenden Anteilen von Methanol und 2 % Triethlamin eluiert. Man erhältso 140 mg der Titelverbindung, die, aus Hexan umkristallisiert, bei 127-130°C schmilzt.

## Beispiel 3

***(E)-3-(1-Imidazolyl)-3-(1-methoxyimino-1,2,3,4-tetrahydro-6-naphthyl)-acrylsäure-tert. butylester***

2,48 g 5-Oxo-5,6,7,8-tetrahydro-2-naphtolsäure (Chem. Parm. Bull. 32 (1984) 130) werden in 20 ml Pyridin mit 1,26 g O-Methylhydroxylamin, Hydrochlorid versetzt. Nach 3 Stunden bei Raumtemperatur wird auf 1 M Salzsäure gegeben, mit Essigester extrahiert, getrocknet ($Na_2SO_4$) und eingedampft. Der kristalline Rückstand wird mit 10 ml Thionylchlorid 15 Minuten bei Raumtemperataur gerührt. Dann wird das Thionylchlorid bei 30°C im Vakuum abdestilliert. Man destilliert noch zweimal mit je 10 ml Methylenchlorid nach, löst den Rückstand in 30 ml Tetrahydrofuran und versetzt mit 1,9 ml N-Trimethylsilylimidazol. Nach 15 Minuten bei Raumtemperatur wird im Vakuum vollständig eingedampft und der Rückstand erneut in 30 ml Tetrahydrofuran gelöst und mit 5,8 g tert. Butoxycarbonyltriphenylphosphoran versetzt. Nach 17 Stunden Kochen am Rückfluß wird auf 1 M Salzsäure gegeben und mit Essigester extrahiert. Die Salzsäurephase wird mit Kaliumcarbonat alkalisiert und mit Essigester extrahiert. Nach Trocknen und Eindampfen der Essigesterphase hinterbleiben 4,1 g Rohprodukt, das an 60 g Kieselgel chromatographiert wird. Mit Methylenchlorid/Methanol (99:1) eluiert man 2,3 g Produkt, aus dem durch Kristallisation 930 mg des reinen E-Isomeren der Titelverbindung erhalten werden; Fp. 128°-135°C.

## Beispiel 4

***(E/Z)-3-(1-Hydroxy-1,2,3,4-tetrahydro-6-naphthyl)-3-(1-imidazolyl)-acrylsäure-tert. butylester***

1,14 g 5-Oxo-5,6,7,8-tetrahydro-2-naphtholsäure in 20 ml Dioxan/Wasser (9:1, V/V) werden unter Eiskühlung mit 800 mg Natriumborhydrid langsam versetzt. Nach einer Stunde Rühren bei Raumtemperatur wird auf 2 M Salzsäure gegeben und mit Essigester extrahiert. Nach Waschen der Essigesterphase mit Kochsalzlösung, Trocknen ($Na_2SO_4$) und Eindampfen erhält man 1,13 g der Hydroxysäure, die in 10 ml Pyridin gelöst und mit 1 ml Essigsäureanhydrid versetzt wird. Nach 4 Tagen bei Raumtemperatur wird Essigester zugegeben, mit eiskalter 1 M Salzsäure dreimal gewaschen und die Essigesterphase getrocknet ($Na_2SO_4$) und eingedampft. Nach Absaugen des kristallinen Rückstandes mit Hexan erhält man 1,2 g 5-Acetoxy-5,6,7,8-tetrahydro-2-naphtholsäure. 1,1 g davon werden mit 10 ml Thionylchlorid eine Stunde bei Raumtemperatur gerührt; dann wird im Vakuum eingedampft und zweimal mit Methylenclorid nachdestilliert.Der Rückstand wird in 10 ml Tetrahydrofuran gelöst und mit 0,73 ml N-Trimethylsilylimidazol versetzt. Die weitere Umsetzung mit 1,9 g tert. Butoxycarbonyltriphenyl-phosphoran (6 Stunden Rückfluß), Aufarbeitung und Chromatographie erfolgt analog Beispiel 3. Man erhält 0,8 g des Acetats der Titelverbindungals farbloses Öl. 700 mg davon werden in 10 ml methanolischer Kaliumhydroxidlösung (10 % G/V) 1 Stunde bei Raumtemperatur belassen. Man gibt auf Wasser, extrahiert mit Essigester, trocknet ($Na_2SO_4$) und dampft ein.

Den Rückstand destilliert man noch zweimal mit Methylenchlorid nach. Den Rückstand chromatographiert man an 20 g Kieselgel. Mit Methylenchlorid/Methanol (99:1) eluiert man 394 mg der Titelverbindung als farbloses Harz.

**Beispiel 5**

***(E/Z)-3-(1-Imidazolyl)-3-(1-oxo-1,2,3,4-tetrahydro-6-naphthyl)-acrylsäure-tert.butylester***

140 mg der in Beispiel 4 beschriebenen Endverbindung werden in 5 ml Methylenchlorid gelöst und mit 800 mg Mangandioxid 18 Stunden bei Raumtemperatur gerührt. Nach Abfiltrieren des Mangandioxids und Eindampfen hinterbleibt ein Roprodukt, das nach Reinigung durch Dickschichtchromathographie (Laufmittel Methylenchlorid/Methanol 9:1) 64 mg der Titelverbindung als dickes Öl ergibt.

**Beispiel 6**

E-3-(1-Methyl-6-benzotriazolyl)-3-(1,2,4-triazol-1-yl)-acrylsäure-tert.-butylester

2,8 g 1-Methyl-benzotriazol-6-carbonsäure (Beispiel 1) werden analog Beispiel 1, jedoch unter Verwendung von 2,35 g 1-Trimethylsilyl-1,2,4-triazol, umgesetzt. Durch chromatographische Trennung an Kieselgel (Laufmittel Dichlormethan/2-Propanol 99:1) wird die Titelverbindung rein erhalten. Fp.: 145-150 °C

**Beispiel 7**

E-und Z-3-(1-Methyl-6-benzimidazolyl)-3-(1-imidazolyl)-acrylsäure-tert.-butylester

1 g 1-Methyl-benzimidazol-6-carbonsäure werden analog Beispiel 1 umgesetzt. Nach Trennung mittels HPLC erhält man das E-Isomer der Titelverbindung (Fp.: 183-185 °C) und das Z-Isomer (Fp.: 69-71 °C) in reiner Form. Die erforderliche 1-Methyl-benzimidazol-6-carbonsäure wird durch PermanganatOxidation von 1,6-Dimethyl-benzimidazol erhalten.

**Beispiel 8**

3-(1-Methyl-6-indazolyl)-3-(1-imidazolyl)-acrylsäure-tert.-butylester

die Titelverbindung wird analog Beispiel 1 aus 1-Methylindazol-6-carbonsäure erhalten.
Die 1-Methylindazol-6-carbonsäure wird aus 1,6-Dimethylindazol durch Permanganat-Oxidation erhalten.

**Patentansprüche**

**1.** Bicyclisch substituierte Vinylimidazole, -triazole und -tetrazole der allgemeinen Formel I

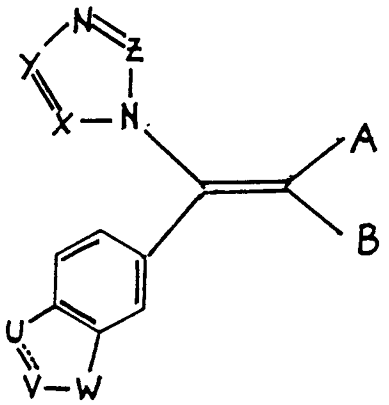

(I),

worin

X, Y und Z gemeinsam mit den beiden Stickstoffatomen einen Imidazol-, Triazol- oder Tetrazolrest bilden, A und B gleich oder verschieden sind und je ein Wasserstoffatom, eine gerad- oder verzweigtkettige $C_1$ - $C_{12}$ -Alkylgruppe

oder eine gegebenenfalls substituierte Arylgruppe

oder entweder

A oder B ein Wasserstoffatom und der jeweils andere Substituent B oder A eine gegebenenfalls veresterte Caboxylgruppe, eine gegebenenfalls substituierte Carbonsäureamidgruppe, eine Alkyl- oder Arylketongruppe oder eine Nitrilgruppe oder A und B gemeinsam einen Cycloalkyl- oder Polycycloalkylrest, die jeweils ein- oder mehrfach durch $C_1$ - $C_8$ - Alkylgruppen oder Halogenatome substituiert sein können, und U entweder den Rest >CH-OH oder die Gruppe >C=D, wobei D für ein Sauerstoffatom oder für einen Rest =N-$OR^1$ sowie -V-W- für einen Alkylenrest $-(CH_2)_n-$ mit n= 2, 3 oder 4 stehen,

oder

U die Gruppe ⋗C-$R^2$, die doppelt an V gebunden ist und wobei V den Rest ⋗C-$R^3$ und W den Rest >N-$R^4$ darstellen, oder U ein Stickstoffatom, das doppelt an V gebunden ist und wobei V dann ebenfalls ein Stickstoffatom oder die Gruppierung ⋗C-$R^5$ und W die Gruppe >N-$R^6$ darstellen mit $R^1$, $R^2$, $R^3$, $R^4$, $R^5$ und $R^6$ jeweils in der Bedeutung eines Wasserstoffatoms oder einer gerad- oder verzweigtkettigen $C_1$ - $C_{12}$ - Alkylgruppe, bedeuten.

**2.** Vinylazole der allgemeinen Formel I, worin X, Y und Z jeweils eine >CH-Gruppe bedeuten.

**3.** Vinylazole der allgemeinen Formel I, worin, wenn $R^1$ oder $R^2$ und/oder $R^3$ und/oder $R^4$ oder $R^5$ und/oder $R^6$ Alkylgruppen bedeuten, diese Alkylgruppen 1 bis 8 Kohlenstoffatome aufweisen.

**4.** Verfahren zur Herstellung der Verbindungen der allgemeinen Formel I, dadurch gekennzeichnet, daß entweder

a) eine Verbindung der allgemeinen Formel II

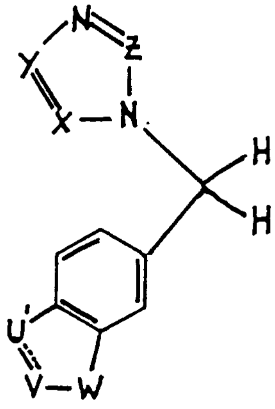

(II),

worin
V, W, X, Y und Z die in Formel I angegebene Bedeutung haben, U' den Rest >CH-OH oder >C = N-OR[1], wobei R[1] die in Formel I angegebene Bedeutung hat und die Hydroxygruppe in U' gegebenenfalls geschützt ist, bedeutet und die H-Atome der die beiden Ringsysteme überbrückenden Methylengruppe acide sind mit einem Keton der allgemeinen Formel III

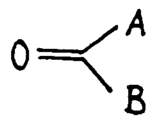

(III),

worin A und B die in Formel I angegebene Bedeutung haben, in Gegenwart einer Base zu einer Verbindung der allgemeinen Formel IV

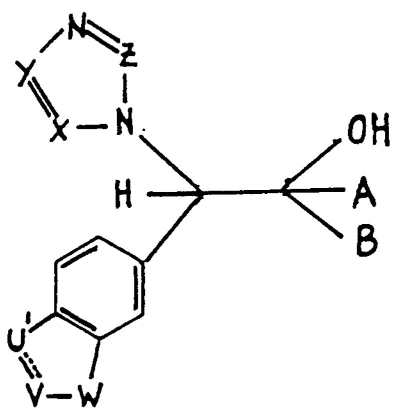

(IV),

umgesetzt und diese mit einem wasserabspaltenden Mittel und nach gegebenenfalls erforderlicher Abspaltung der Schutzgruppe in U' in eine Verbindung der allgemeinen Formel I umgewandelt oder
b) eine Verbindung der allgemeinen Formel V

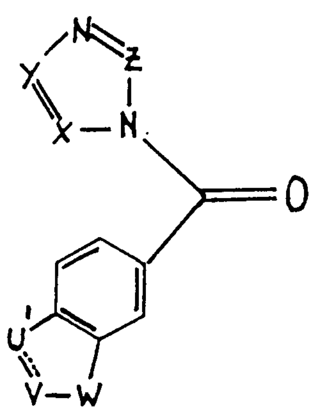

(V),

worin
V, W, X, Y und Z die in Formel I angegebene Bedeutung haben und U' für den Rest >CH-OH oder >C = N-OR[1], wobei R[1] die in Formel I angegebene Bedeutung hat mit einem Wittig-Reagens der allgemeinen Formel VI

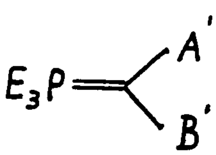

(VI),

worin A' oder B' ein Wasserstoffatom und der jeweils andere Substituent B' oder A' eine gegebenenfalls veresterte Carboxylgruppe, eine gegebenenfalls substituierte Carbonsäureamidgruppe, eine Alkyl- oder Alylketongruppe oder eine Nitrilgruppe sowie E einen gegebenenfalls mit einem oder mehreren gerad- oder verzweigtkettigen $C_1$ - $C_6$-Alkylresten oder Halogenatomen substituierten Phenylrest oder einen gerad- oder verzweigtkettigen $C_1$-$C_6$-Alkylrest bedeuten, in einem inerten Lösungsmittel zwischen Raumtemperatur und Siedetemperatur des verwendeten Lösungsmittel umgesetzt

oder

c) eine Verbindung der allgemeinen Formel VII

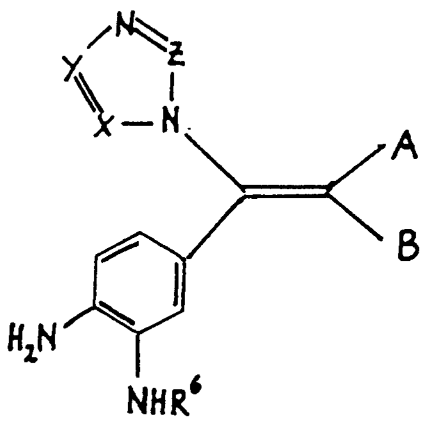

(VII)

worin A, B, X, Y, Z und $R^6$ die in Formel I angegebene Bedeutung haben, in an sich bekannter Weise mit einer Säure $R^5$-COOH oder einem Aldehyd $R^5$-CHO, worin $R^5$ die in Formel I angegebene Bedeutung hat, im Falle des Aldehyds unter milde oxidierenden Bedingungen, oder mit salpetriger Säure oder deren Estern umgesetzt wird.

**5.** Pharmazeutische Präparate, die mindestens eine Verbindung gemäß den Ansprüchen 1 bis 3 sowie einen pharmazeutisch verträglichen Träger enthalten.

**6.** Verwendung der Verbindungen gemäß den Ansprüchen 1 bis 3 zur Herstellung von Arzneimitteln.

Europäisches Patentamt

# EUROPÄISCHER RECHERCHENBERICHT

Nummer der Anmeldung

EP 92 25 0152

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | WO-A-9 101 975 (SCHERING AG)<br>*Insgesamt*<br>& EP-A-0 411 735 (SCHERING AG) | 1-6<br><br>1-6 | C07D403/06<br>C07D233/60<br>C07D249/18<br>C07D235/06<br>A61K31/395<br>A61K31/41<br>C07D249/04<br>C07D249/08 |
| A | EP-A-0 227 100 (SHIONOGI SEIYAKU KABUSHIKI KAISHA)<br>*Insgesamt* | 1-6 | |
| D,A | EP-A-0 236 940 (CIBA-GEIGY)<br>*Insgesamt* | 1-6 | |
| D,A | EP-A-0 165 777 (ELI LILLY)<br>* Seite 32, Zeile 22 - Zeile 23; Anspruch 1 * | 1-6 | |
| | | | **RECHERCHIERTE SACHGEBIETE (Int. Cl.5 )** |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 29 SEPTEMBER 1992 | LUYTEN H.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)